# EUROPEAN PATENT SPECIFICATION

(11) **EP 2 384 215 B1**
(45) Date of publication and mention of the grant of the patent: **10.04.2013**
(21) Application number: 09797177.4
(22) Date of filing: 09.12.2009
(51) Int. Cl.: A61M 16/06, A61M 16/20

(54) **Mask for delivering a therapeutic breathable substance**
Maske zur Abgabe einer therapeutischen einatembaren Substanz
Masque pour distribuer une substance thérapeutique respirable

(30) Priority: 30.12.2008 US 141254 P
(43) Date of publication of application: 09.11.2011
(73) Proprietor: Koninklijke Philips Electronics N.V., 5621 BA Eindhoven (NL)
(72) Inventor: VON HOLLEN, Dirk, Briarcliff Manor, New York 10510-8001 (US); DENYER, Jonathan, Stan, Briarcliff Manor, New York 10510-8001 (US)
(74) Representative: Van Velzen, Maaike Mathilde
(86) International application number: PCT/IB2009/055634
(87) International publication number: WO 2010/076715

(56) References cited:
- EP-A2- 1 854 493
- WO-A1-2005/099798
- US-A- 5 730 122

## Description

This application is related to U.S. Patent Application Serial No., 61/141,253 filed December 30, 2008.

The invention relates to the delivery of a therapeutic breathable substance to the airway of a subject.

Conventional nebulizers, spacers, and other therapeutic gas delivery mechanisms implement masks that enclose the nostrils and/or mouth of a subject for delivery of a breathable substance. Generally, these masks include exhaust valves for exhausting gas exhaled by the subject to atmosphere. These valves are typically formed on the body of the mask, which may impede a view of the nose and/or mouth of the subject through the mask for a caregiver monitoring the therapy. Conventional masks may not provide an adequate indicator to the monitoring caregiver of whether or not the subject has exhaled. This may be important for therapies that are dosed in terms of breaths. US5,730,122 discloses a facial mask including a one-way expiration valve and an inspiratory threshold valve.

One aspect of the invention relates to a mask for substantially sealing the airway of a subject for delivery of a breathable substance as described in claim 1. The mask comprises a mask body, a source connector, a mask seal portion, and a one-way valve. The mask body is configured to enclose one or more external orifices of the airway of a subject, and forms a substance opening through which a breathable substance can be communicated to the one or more external orifices of the subject that are enclosed by the mask body. The source connector is disposed on the mask body, and comprises a conduit that forms a gas flow path between the substance opening of the mask body and a source interface opening formed in the source connector. The source connector is configured to connect a source of the breathable substance to the gas flow path between the substance opening of the mask body and the source interface opening. The mask seal portion is attached to the mask body, and is configured to provide a substantially sealed engagement between the face of the subject and the mask body around the one or more external orifices of the airway of the subject. The one-way valve is disposed on the source connector, and is configured to permit gas within the gas flow path formed by the source connector to be exhausted to ambient atmosphere and to substantially seal the gas flow path from inflows of ambient atmosphere.

An illustrative aspect relates to a mask for substantially sealing the airway of a subject for delivery of a breathable substance. In said aspect, the mask comprises means for forming a chamber that encloses one or more external orifices of the airway of a subject, wherein a portion of the chamber is formed by the face of the subject; means for placing a source of a breathable substance in communication with the chamber via a conduit forming a gas flow path between a source interface opening and a substance opening formed in the chamber by connecting the source of the breathable substance to the source interface opening; means for delivering the breathable substance from the source of the breathable substance to the chamber through the conduit without permitting inflows from ambient atmosphere into the conduit; and means for exhausting gas exhaled by the subject to ambient atmosphere through an exhaust opening in the conduit.

Another illustrative aspect relates to a mask for substantially sealing the airway of a subject for delivery of a breathable substance. In said aspect, the mask comprises a mask body, one or more exhaust openings formed in the mask body, a first valve member, and a second valve member. The mask body is configured to enclose one or more external orifices of the airway of a subject such that the mask body and the face of the subject form a chamber around the one or more external orifices of the airway of the subject that are enclosed by the mask body. The mask body further includes a source connector configured to connect a source of a breathable substance with the chamber such that a flow of the breathable substance is provided from the source to the chamber through the source connecter. The one or more exhaust openings formed in the mask body exhaust gas within the chamber to ambient atmosphere. The first valve member covers a portion of the one or more exhaust openings to block inflows of gas at ambient atmosphere from entering the chamber via the one or more exhaust openings. The first valve member is configured such that exhalation of gas from the one or more external orifices of the airway of the subject applies a force that actuates the first valve member to uncover the portion of the one or more exhaust openings covered by the first valve member in order to exhaust the exhaled gas if the pressure of exhaled gas within the chamber is equal to or greater than a first pressure. The second valve member covers a portion of the one or more exhaust openings to block inflows of gas at ambient atmosphere from entering the chamber via the one or more exhaust openings, and is configured such that exhalation of gas from the one or more external orifices of the airway of the subject applies a force that actuates the second valve member to uncover the portion of the one or more exhaust openings covered by the second valve member if the pressure of exhaled gas within the chamber is equal to or greater than a second pressure. The first pressure is less than the second pressure such that actuation of the first valve member by exhaled gas provides an indicator to a caregiver of relatively weak exhalations by the subject and actuation of the second valve member by exhaled gas occurs in combination with actuation of the first valve member during stronger exhalations by the subject to reduce the resistance to gas flow through the one or more exhaust openings during relatively strong exhalations.

Another illustrative aspect relates to a mask configured to substantially seal the airway of a subject for delivery of a breathable substance. In one embodiment the mask comprises means for forming a chamber that encloses one or more external orifices of the airway of a subject, wherein a portion of the chamber is formed by the face of the subject; means for blocking inflows of gas at ambient atmosphere from entering the chamber via one or more exhaust openings, wherein the means for blocking comprise a first valve member that covers a portion of the one or more exhaust openings and a second valve member that covers a portion of the one or more exhaust openings; means for, at or above a first pressure within the chamber, uncovering the portion of the one or more exhaust openings covered by the first valve member to release gas within the chamber to atmosphere; means for, at or above a second pressure within the chamber, uncovering the portion of the one or more exhaust openings covered by the second valve member to release gas within the chamber to atmosphere; wherein the first pressure is less than the second pressure such that the uncovering of the portion of the one or more exhaust openings covered by the first valve member to release exhaled gas to atmosphere provides an indicator to a caregiver of relatively weak exhalations by the subject and the uncovering of the portion of the one or more exhaust openings covered by the second valve member to release exhaled gas occurs in combination with the uncovering of the portion of the one or more exhaust openings covered by the first valve member during stronger exhalations by the subject to reduce the resistance to gas flow through the on or more exhaust openings during relatively strong exhalations.

Objects, features, and characteristics of the present invention, as well as the methods of operation and functions of the related elements of structure and the combination of parts and economies of manufacture, will become more apparent upon consideration of the following description and the appended claims with reference to the accompanying drawings, all of which form a part of this specification, wherein like reference numerals designate corresponding parts in the various figures. In one embodiment of the invention, the structural components illustrated herein are drawn to scale. It is to be expressly understood, however, that the drawings are for the purpose of illustration and description only and are not a limitation of the invention. In addition, it should be appreciated that structural features shown or described in any one embodiment herein can be used in other embodiments as well insotar as they are covered by the claims. As used in the specification and in the claims, the singular form of "a", "an", and "the" include plural referents unless the context clearly dictates otherwise.

FIG. 1 illustrates a mask 10 for use in therapeutic gas delivery, in accordance with one or more embodiments of the invention.

FIG. 2 illustrates a mask 10 for use in therapeutic gas delivery, in accordance with one or more embodiments of the invention.

FIG. 3 illustrates a mask 10 for use in therapeutic gas delivery, in accordance with one or more illustrative embodiments.

FIG. 4 illustrates a mask 10 for use in therapeutic gas delivery, in accordance with one or more embodiments of the invention.

FIG. 5 illustrates a method of isolating the airway of a subject from atmosphere to facilitate delivery of a breathable substance.

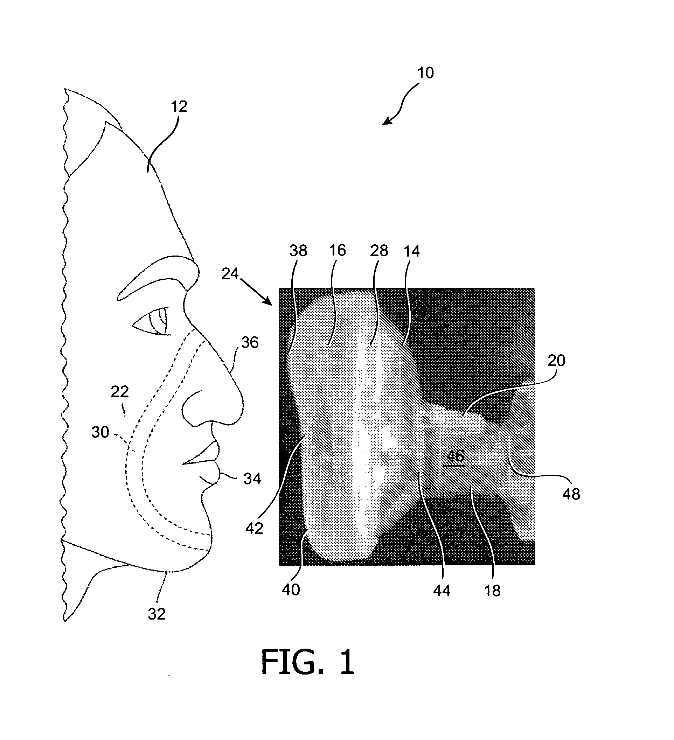

FIG. 1 illustrates a mask 10 for use in therapeutic gas delivery to one or more external orifices (e.g., the nostrils and/or mouth) of a subject 12 in accordance with the present invention. The mask 10 may generally include a mask body 14, a seal portion 16, a source connector 18, and an exhaust valve 20. The mask 10 is configured to be placed against the face 22 of subject 12 with a substantially sealed interface therebetween such that a chamber 24 is formed by mask 10 and the face of subject 12 that encloses the one or more external orifices of subject 12. During use, a breathable substance can be provided into chamber 24 from a source (not shown) of the breathable substance through source connector 18.

Mask body 14, in one embodiment, is made of a relatively more rigid material (higher durometer material) than seal portion 16. For example, mask body 14 may be made from polycarbonate, or other suitable material. The mask body 14 provides structural rigidity to the mask 10 and, in some embodiments, may be a portion of a disposable mask that is retained when replacing the seal portion 16. The mask body 14/seal portion structure may be formed by a two-step molding or assembly process. For example, the relatively harder mask body 14 may be molded first and then inserted into a second mold for the seal portion 16, which is injection molded to form around and/or into the mask body 14. In one embodiment, mask body 14 and seal portion 16 are of a similar durometer (e.g., less rigid), and/or may be formed as a single piece.

Seal portion 16 may be made of a relatively soft and/or flexible material so that the seal portion 16 conforms to the shape of the face 22 of subject 12 when held against it. Seal portion 16 may be made of, for example, silicone or an elastomeric material. In one embodiment, seal portion 16 has an arcuate or partially tubular cross-sectional configuration (also referred to as a concavo-convex cross-section, having a generally concave inner surface and generally convex outer surface). In one embodiment, seal portion 16 has a generally rounded, convex face engaging surface for engaging face 22 of subject 12. In one embodiment, the face engaging surface has a more flattened, convex configuration. Also, different regions of seal portion 16 around the perimeter of mask body 14 may have different cross-sectional configurations. Various other seal portion configurations will become apparent to those skilled in the art.

In one embodiment, seal portion 16 is attached to mask body 14 at an attachment region 28, as shown in FIG. 1. Attachment region 28 may be along the perimeter or edge of the mask body 14 and along a perimeter or edge of the seal portion 16, such that there is some overlap of the respective edges of the mask body 14 and the seal portion 16, as shown. Accordingly, there is some material of the mask body 14 that engages with some material of seal portion 16, such that a layered connection is formed. Other embodiments are contemplated in which there is no overlap, such as by attaching mask body 14 and seal portion 16 with their edges end to end (e.g., by an adhesive connection), and/or where mask body 14 and seal portion 16 are formed as a single piece.

Mask body 14 and seal portion 16 are configured to engage face 22 of subject 12 in an engagement region 30 that extends from between the chin 32 and lips 34, upwardly along region 30 on opposite sides of the mouth of subject 12, and across the nose 36 and over the bridge of the nose 36, as shown. In this embodiment, seal portion 16 is generally oblong or pear shaped, as shown in FIG. 1. Seal portion 16 accordingly includes an upper portion 38, a lower portion 40, and a transition portion 42. Upper portion 38, which may be configured to engage the face 22 of subject 12 across the bridge of the nose 36, has a radius of curvature that is relatively smaller than the radius of curvature R2 of lower portion 40. Lower portion 40, with its comparatively larger radius of curvature, may be configured to engage the face 22 of subject 12 between chin 32 and lips 34. Transition portion 42 extends generally from upper portion 38 to lower portion 40 and engages the face 22 of subject 12 and beneath the eyes. To facilitate creating a sealed engagement with face 22 of subject 12, transition portion 42 of seal portion 16 has more material in its cross-section, and has a greater linear length (i.e., if seal portion 16 were to be unrolled or straightened into a linear configuration) when measured in a direction extending orthogonally away from mask body 14, than each of upper portion 38 and lower portion 40, so as to effectively block gas within chamber 24 from reaching the eyes of subject 12.

While the embodiment of seal portion 16 and mask body 14 may be sized to enclose the mouth and nose 36 of subject, this is not intended to be limiting. In one embodiment, mask 10 may be configured to enclose fewer external orifices of the airway of subject 12 (e.g., only the nostrils, only one nostril, only the mouth, etc.). One of ordinary skill in the art will appreciate that the configuration of mask 10 may vary and is not limited to a particular size or configuration, as subject 12 may range in age, size, and/or medical treatment required so as to require appropriate selection from among a variety of different mask sizes and configurations.

On a side of the mask body 14 that is opposite from seal portion 16, mask body 14 forms an opening 44 for receipt of source connector 18. Through opening 44, a breathable substance is provided into chamber 24 for inhalation by subject 12. Also, gas exhaled by subject 12 may be exhausted from chamber 24 to ambient atmosphere via opening 44. In one embodiment, opening 44 is configured to connect with source connector 18.

In one embodiment, source connector 18 comprises a conduit 46 that forms a gas flow path, when connected with mask body 14, from opening 44 to a source interface opening 48 formed in source connector 18. Source connector 18 is configured to connect a source of a breathable substance to the gas flow path between opening 44 of mask body 14 and source interface opening 48 so that the breathable substance can be delivered from source interface opening 48 to chamber 24 for inhalation by subject 12. In one embodiment, source connector 18 is formed from a relatively rigid material (*e.g.*, rigid silicone resin). In one embodiment, source connector 18 is formed from a less rigid material (*e.g*., flexible silicone). In one embodiment, conduit 46 of source connector 18 formed separately from mask body 14, and is securely attached to mask body 14. For example, source connector 18 may be securely attached to opening 44 formed in mask body 14 by a permanent snap fit, an adhesive, heat staking, ultra-sonic welding, and/or otherwise secured to mask body 14.

In one embodiment, source connector 18 is configured to selectably and releasably engage the source of the breathable substance. In one embodiment, source connector 18 is configured to be permanently attached to the source and/or to be formed integrally with the source. The source of the breathable substance may include one or more of metered dose inhaler, a pressurized metered dose inhaler, a static chamber in communication with an inhaler, a nebulizer, a spacer, an aerosol dispenser, and/or other suitable sources of breathable substances. It will be appreciated that, in order to appropriately interface with mask body 14 and the source of the breathable substance, source connector 18 can be of various sizes, shapes, and configurations other than the ones illustrated in FIG. 1.

Exhaust valve 20 is disposed on source connector 18, and is configured to exhaust gas exhaled from the enclosed one or more external orifices of subject 12 to ambient atmosphere. However, exhaust valve 20 is further configured to substantially seal the gas flow path formed within source connector 18 from inflows of ambient atmosphere. As such, exhaust valve 20 is a one-way valve.

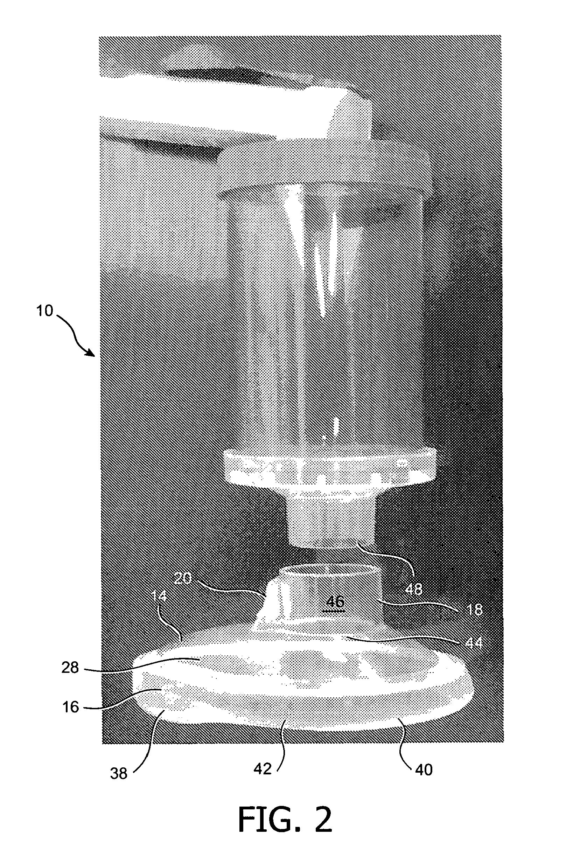

FIG. 2 illustrates how mask 10 can be removed from the source. In the embodiment illustrated in FIG. 2, the source is a spacer that is connected to a metered dose inhaler. The metered dose inhaler is configured to dispense a breathable substance into the spacer, and the breathable substance is received by source connector 18 (when connected) from the spacer/metered dose inhaler combination. In the embodiment illustrated in FIG. 2, the selectively removable attachment of source connector 18 to the spacer is accomplished via a friction fit.

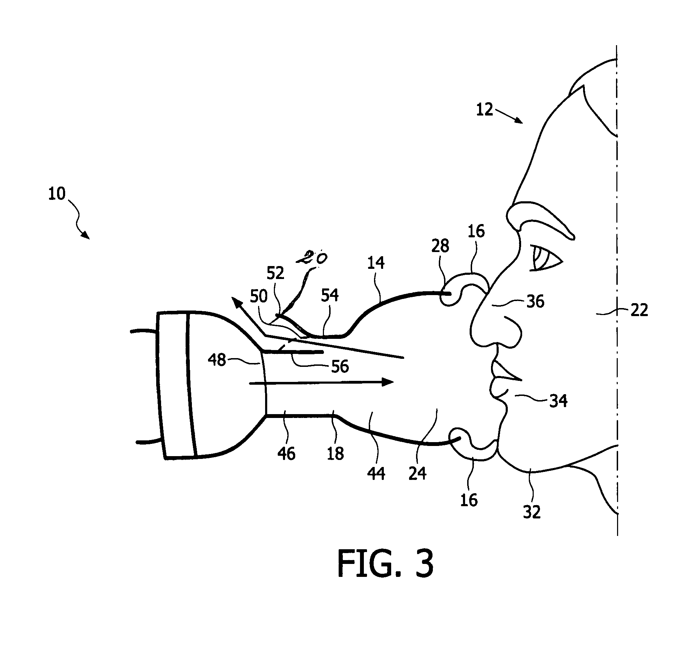

FIG. 3 shows a cross-sectional view of mask 10, in accordance with illustrative embodimens not within the scope of the invention. In the view shown in FIG. 3, seal portion 16 has been engaged with face 22 of subject 12 to form chamber 24 between the face 22 and mask body 14. During inhalation by subject 12, air intermixed with the breathable substance is received into chamber 24 via source connector 18 and opening 44. During exhalation by subject 12, the pressure within chamber 24 opens exhaust valve 20, thereby releasing exhaled gas from chamber 24 to ambient atmosphere.

In the embodiment shown in FIG. 3, exhaust valve 20 includes an exhaust opening 50 formed in conduit 46 and a valve member 52. The default position of valve member 52 is covering exhaust opening 50 to substantially seal conduit 46 from inflows of gas at ambient atmosphere. In one embodiment, valve member 52 is biased into this default position by one or more of a spring bias, a magnetic force, gravity, a resiliency of the materials of valve member 52, and/or other forces. In one embodiment, valve member 52 is only attached to source connector 18 along an attached side 54. If the pressure within chamber 24 reaches a predetermined level, valve member 52 is actuated away from exhaust opening 50 to release gas within chamber 24 to atmosphere. In one embodiment, to actuate away from exhaust opening 50, valve member 52 pivots at or near attached side 54. The pivoting of valve member 52 at or near attached side 54 may be due to a hinge provided at or near attached side 54. In one embodiment, to actuate away from exhaust opening 50, valve member 52 resiliently flexes away from exhaust opening 50.

The disposition of exhaust valve 20 on source connector 18, rather than on mask body 14, may provide one or more enhancements to the functionality of mask 10. For example, generally, mask body 14 is formed from a transparent or translucent material to enable a caregiver to visually monitor the reception of treatment by subject 12. The caregiver may monitor, for instance, the enclosed one or more external orifices to for indications as to the efficacy of the treatment being received and/or for indications that subject 12 is being ventilated adequately during respiration. By way of non-limiting example, the caregiver may check to see if the mouth of subject 12 is open and/or if the nostrils of subject 12 are flared during inspiration, indicating a certain amount of effort being expended in order to inhale. As another example, the caregiver may monitor the color of lips 34 of subject 12, as lips that become purple or blue may indicate that subject 12 is not being adequately ventilated. By locating exhaust valve 20 on source connector 18, the caregiver is provided with a substantially unimpeded view of the mount and/or nose of subject 12 during treatment.

In one embodiment, the uncovering of exhaust opening 50 provides an indicator to the caregiver of exhalation by subject 12. In the embodiment illustrated in FIG. 3, pressure within chamber 24 resulting from exhalation by subject 12 causes valve member 52 to be actuated away from exhaust opening 50 to uncover exhaust opening and exhaust exhaled gas to ambient atmosphere. As should be appreciated from the illustration provided in FIG. 3 and the description above, actuation of valve member 52 may include valve member 52 pivoting or resiliently bending at or near attached side 54. This pivoting or bending would be observable for the caregiver and would provide a marker to the caregiver that a breath has been taken by subject 12. The implementation of a hinge at or along attached side 54 may, in some instances, increase the range of motion of valve member 52, which enhances the visible marker provided by valve 20 of exhalation by subject 12. For treatments administered through mask 10 that are given for a predetermined number of breaths (e.g., treatments from a metered dose inhaler, etc.), providing the caregiver with a mechanism for marking and/or counting breaths will facilitate administration of treatment.

It will be appreciated that in embodiments in which the actuation of valve member 52 away from exhaust opening 50 provides a marker to the caregiver that a breath has been taken by subject 12 there is a tension between at least two design values of exhaust valve 20. The first design value is the sensitivity of the marker provided to the caregiver of exhalation, and the second design value is the reduction of resistance to exhaled gas associated with exhaust valve 20. In order to be adequately sensitive to exhalations, particularly for children, elderly adults, and/or other subjects that are particularly weak, the opening size of exhaust opening 50 may be reduced. This will increase the pressure within chamber 24 during exhalation, the flow rate of gas through exhaust opening 50, and the corresponding force applied by the gas to valve member, all of which will enhance the sensitivity of the marker to exhalation. However, the increase in pressure within chamber 24 during exhalation caused by the increase in resistance of exhaust opening 20 caused by a reduction in the size of exhaust opening 50 may be associated with one or more drawbacks. These drawbacks may include a discomfort of subject 12 (particularly for stronger subjects) caused by the increased pressure, back pressure, and/or resistance to exhalation, and/or other drawbacks.

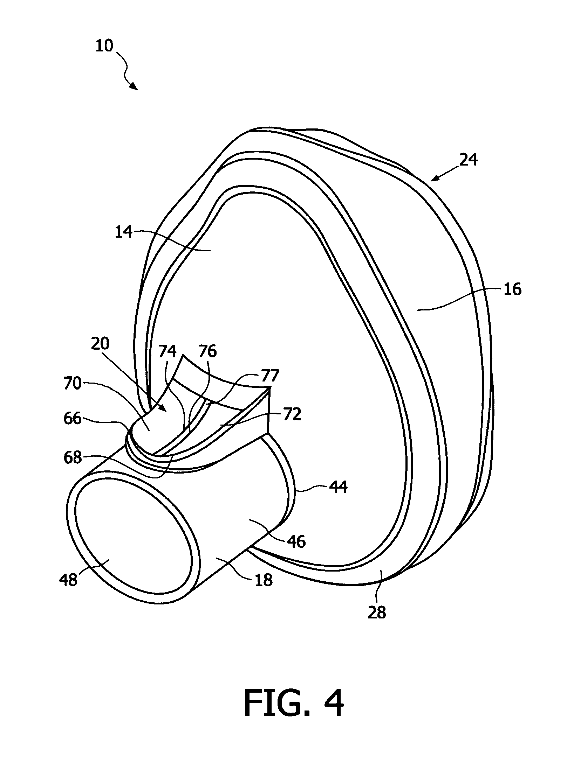

FIG. 4 illustrates an embodiment of mask 10 according to the invention which the sensitivity of the marker of exhalation provided by exhaust valve 20 is enhanced while reducing the resistance of exhaust valve 20 during exhalation for stronger subjects. In the embodiment, shown in FIG. 4, exhaust valve 20 includes two separate exhaust openings formed in source connector 18, a first exhaust opening 66 and a second exhaust opening 68, which are covered by a first valve member 70 and a second valve member 72, respectively.

In one embodiment, the area of first exhaust opening 66 is larger than second exhaust opening 68. The outer perimeter of first valve member 70 corresponds to the edge of first exhaust opening 66 such that first valve member 70 has a default position at which first valve member 70 covers first exhaust opening 66 to block inflows of gas through first exhaust opening 66 from ambient atmosphere into the chamber formed by mask body 14 and the face of the subject. Similarly, the outer perimeter of second valve member 72 corresponds to the edge of first exhaust opening 68 such that second valve member 72 has a default position at which second valve member 72 covers second exhaust opening 68 to block inflows of gas from ambient atmosphere through second exhaust opening 68. First valve member 70 and second valve member 72 are attached to source connector at attached edges 74 and 76, respectively. First valve member 70 and second valve member 72 are configured to pivot or resiliently bend at or near attached edges 74 and 76 to actuate away from first exhaust opening 66 and second exhaust opening 68. In one embodiment, one or both of first valve member 70 and second valve member 72 are attached to exhaust valve 20 by a hinge disposed along attached side 74 and/or attached side 76.

In one embodiment, at or above a first pressure within the chamber formed by mask body 14 and the face of the subject, first valve member 70 is actuated away from first exhaust opening 68 to exhaust gas from within the chamber to atmosphere, thereby providing a marker of exhalation of the subject. At or above a second pressure that is greater than the first pressure, second valve member 72 is actuated away from second exhaust opening 68 to exhaust gas from within the chamber to atmosphere, thereby reducing the resistance of exhaust valve 20 to gas flowing out of the chamber through exhaust valve 20. Alternatively and/or additionally to providing a relative size differentials of first exhaust opening 22 and second exhaust opening 68, the relative thickness and/or weight of first valve member 70 and second valve member 72 may be different in order to further tailor the pressure differential between the first pressure and the second pressure, and/or to ensure that first valve member 70 provides an adequate marker for exhalalations.

In one embodiment, first valve member 70 and second valve member 72 are formed from separate pieces of material. In one embodiment, first valve member 70 and second valve member 72 are formed from a single piece of material with a cross-member 77 providing an anchor that separates first valve member 70 from second valve member 72. Cross-member 77 is a rigid structure that holds the piece of material forming valve members 70 and 72 in place along attached edges 74 and 76.

It will be appreciated that the embodiments of exhaust valve 20 shown in FIG. 4 that enhances the sensitivity of the marker of exhalation provided by exhaust valve 20 while reducing the resistance of exhaust valve 20 during stronger and/or more voluminous exhalation are not intended to be limiting. The scope of the disclosure of this feature encompasses any embodiments of exhaust valve 20 in which a first portion one or more exhaust openings are uncovered to exhaust gas from within the chamber formed between mask body 14 and the face of a subject if the pressure within the chamber is greater than or equal to a first, relatively low pressure, and a second portion of the one or more exhaust openings are uncovered to exhaust gas if the pressure within the chamber is greater than or equal to a second pressure that is higher than the first pressure. For example, a plurality of valve members may cover a single exhaust opening. As another example, the valve members covering the one or more exhaust openings may or may not be flaps (*e.g*., attached along one edge). The number of valve members and/or exhaust openings included in exhaust valve 20 may be greater than two. The exhaust openings of exhaust valve 20 may be located separately from each (*e.g.*, not adjacent as shown in FIG. 4). Exhaust valve 20 may include one or more exhaust openings formed on mask body 14, rather than on source connector 18.

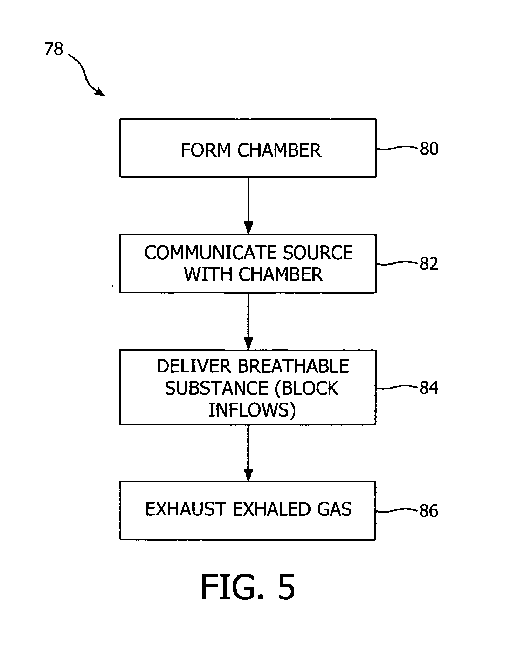

FIG. 5 illustrates a method 78 of isolating the airway of a subject from atmosphere to facilitate delivery of a breathable substance. The operations of method 78 presented below are intended to be illustrative. In some embodiments, method 78 may be accomplished with one or more additional operations not described, and/or without one or more of the operations discussed. Additionally, the order in which the operations of method 78 are illustrated in FIG. 5 and described below is not intended to be limiting. Although the operations of method 78 are described below with references to components of a mask that is the same as or similar to mask 10 (shown in FIGS. 1-4 and described above), this is not intended to be limiting. Method 78 may be implemented in a variety of other contexts without departing from the scope of this disclosure.

At an operation 80, a chamber is formed that encloses one or more external orifices of the airway of the subject. The chamber is formed in part by the face of the subject. In one embodiment, operation 80 is performed by a mask body that is the same as or similar to mask body 14 (shown in FIGS. 1-4 and described above).

At an operation 82, a source of a breathable substance is placed in communication with the chamber via a conduit. The conduit forms a gas flow path between a source interface opening configured to connect with the source and a substance opening formed in the chamber. In one embodiment, operation 82 is performed by a source connector that is the same as or similar to source connector 18 (shown in FIGS. 1-4 and described above).

At an operation 84, the breathable substance is delivered from the source of the breathable substance to the chamber without permitting inflows from ambient atmosphere into the chamber and/or the conduit. This may include blocking inflows of gas at ambient atmosphere from entering the chamber via one or more exhaust openings. The one or more exhaust openings may include one or more exhaust openings formed in the conduit and/or in the chamber. In one embodiment, operation 84 is performed by an exhaust valve that is similar to or the same as exhaust valve 20 (shown in FIGS. 1-4 and described above).

At an operation 86, gas exhaled by the subject is exhausted to ambient atmosphere through the one or more exhaust openings. In one embodiment, operation 86 includes uncovering a first portion of the one or more exhaust openings at or above a first pressure within the chamber and/or uncovering a second portion of the one or more exhaust openings at or above a second pressure within the chamber that is greater than the first pressure. In one embodiment, operation 86 is performed by an exhaust valve that is the same as or similar to exhaust valve 20 (shown in FIGS. 1-4 and described above).

Although the invention has been described in detail for the purpose of illustration based on what is currently considered to be the most practical and preferred embodiments, it is to be understood that such detail is solely for that purpose and that the invention is not limited to the disclosed embodiments, but, on the contrary, is intended to cover modifications and equivalent arrangements that are within the scope of the appended claims. For example, it is to be understood that the present invention contemplates that, to the extent possible, one or more features of any embodiment can be combined with one or more features of any other embodiment.

## Claims

1. A mask (10) for substantially sealing the airway of a subject (12) for delivery of a breathable substance, the mask (10) comprising:
a mask body (14) configured to enclose one or more external orifices of the, airway the subject (12), wherein the mask body (14) the face (22) of the subject (12) form a chamber (24) around the one or more external orifices of the airway of the subject that are enclosed by the mask body (14) the mask body (14) further including a source connector (18) configured to connect a source of a breathable substance with the chamber (24) such that a flow of the breathable substance is provided from the source to the chamber (24) through the source connector .(18)
one or more exhaust openings (66, 68) formed in the mask body (14) that exhaust gas within the chamber (24) to ambient atmosphere;
a first valve member (70) that covers a portion of the one or more exhaust openings (166,68) block inflows of gas at ambient atmosphere from entering the chamber (24) via the one or more exhaust openings (66,68) the first valve member (70) being configured such that exhalation of gas from the one or more external orifices of the airway of the subject (12) applies a force that actuates the first valve member (70) to uncover the portion of the one or more exhaust openings (66,68) covered by the first valve member (70) in order to exhaust the exhaled gas, wherein the first valve member (70) is actuated to uncover the portion of the one or more exhaust openings (66,68) covered by the first valve member (70) if the pressure of exhaled gas within the chamber (24) equal to or greater than a first pressure; and
a second valve member (72) that covers a portion of the one or more exhaust (24), openings (66,68) to block inflows of gas at ambient atmosphere from entering the chamber (24) via the one or more exhaust openings (66,68) the second valve member (72) being configured such that exhalation of gas from the one or more external orifices of the airway of the subject (12) applies a force that actuates the second valve member (72) to uncover the portion of the one applies a force that actuates the second valve member (72) to uncover the portion of the one or more exhaust openings (66,68) covered by the second valve member, (72) wherein the second valve member (72) is actuated to uncover the portion of the one or more exhaust openings (66,68) covered by the second valve member (72) if the pressure of exhaled gas within the chamber (24) is equal to or greater than a second pressure;
wherein the first pressure is less than the second pressure such that actuation of the first valve member (70) by exhaled gas provides an indicator to a caregiver of relatively weak exhalations by the subject (12) and actuation of the second valve member (72) by exhaled gas occurs in combination with actuation of the first valve member (70) during stronger exhalations by the subject (12) to reduce the resistance to gas flow through the one or more exhaust openings (66,68) during relatively strong exhalations.

2. The mask (10) of claim 1, wherein the resistance of the mask (10) to the flow of exhaled gas to atmosphere is reduced significantly at the second pressure by the actuation of the second valve member (72).

3. The mask (10) of claim 1, wherein the first valve member (70) and the second valve member (72) cover portions of the same exhaust opening (50).

4. The mask (10) of claim 1, wherein one or both of the first valve member (70) and/or the second valve member (72) are a flap.

5. The mask (10) of claim 1, wherein one or both of the first valve member and/or the second valve member (70, 72) are actuated to uncover portions of the one or more exhaust openings (66, 68) by pivoting.

6. The mask (10) of claim 1, wherein the one or more exhaust openings are formed on the source connector (18) of the mask body (14).

## Patentansprüche

1. Maske (10) zum erheblichen Abdichten der Atemwege einer Person (12) zur Abgabe einer einatembaren Substanz, wobei die Maske (10) Folgendes umfasst:
einen Maskenkörper (14), der konfiguriert ist, um eine oder mehrere äußere Öffnungen der Atemwege der Person (12) zu umschließen, wobei der Maskenkörper (14) und das Gesicht (22) der Person (12) eine Kammer (24) um die eine oder mehrere äußere Öffnungen der Atemwege der Person (12) bilden, die durch den Maskenkörper (14) umschlossen sind, wobei der Maskenkörper (14) weiterhin einen Quellenkonnektor (18) umfasst, der konfiguriert ist, um eine Quelle einer einatembaren Substanz mit der Kammer (24) auf derartige Weise zu verbinden, dass ein Strom der einatembaren Substanz von der Quelle durch den Quellenkonnektor (18) zu der Kammer (24) geschaffen wird;
eine oder mehrere in dem Maskenkörper (14) ausgebildete Abluftöffnungen (66, 68), die das in der Kammer (24) befindliche Gas in die umgebende Atmosphäre ableiten;
ein erstes Ventilelement (70), das einen Teil der einen oder mehreren Abluftöffnungen (66, 68) abdeckt, um zu verhindern, dass ein Gasstrom bei umgebender Atmosphäre über die eine oder mehrere Abluftöffnungen (66, 68) in die Kammer (24) eintritt, wobei das erste Ventilelement (70) derartig konfiguriert ist, dass das Ausatmen von Gas aus der einen oder mehreren äußeren Öffnungen der Atemwege der Person (12) eine Kraft ausübt, die das erste Ventilelement (70) betätigt, um den Teil der einen oder mehreren Abluftöffnungen (66, 68), der durch das erste Ventilelement (70) abgedeckt wird, freizulegen, so dass das ausgeatmete Gas abgeleitet wird, wobei das erste Ventilelement (70) betätigt wird, um den Teil der einen oder mehreren Abluftöffnungen (66, 68) , der durch das erste Ventilelement (70) abgedeckt ist, freizulegen, wenn der Druck des ausgeatmeten Gases innerhalb der Kammer (24) einem ersten Druck entspricht oder größer ist als dieser; und
ein zweites Ventilelement (72), das einen Teil der einen oder mehreren Abluftöffnungen (66, 68) abdeckt, um zu verhindern, dass ein Gasstrom bei umgebender Atmosphäre über die eine oder mehrere Abluftöffnungen (66, 68) in die Kammer (24) eintritt, wobei das zweite Ventilelement (72) derartig konfiguriert ist, dass das Ausatmen von Gas aus der einen oder mehreren äußeren Öffnungen der Atemwege der Person (12) eine Kraft ausübt, die das zweite Ventilelement (72) betätigt, um den Teil der einen oder mehreren Abluftöffnungen (66, 68), der durch das zweite Ventilelement (72) abgedeckt wird, freizulegen, wobei das zweite Ventilelement (72) betätigt wird, um den Teil der einen oder mehreren Abluftöffnungen (66, 68), der durch das zweite Ventilelement (72) abgedeckt wird, freizulegen, wenn der Druck des ausgeatmeten Gases innerhalb der Kammer (24) einem zweiten Druck entspricht oder größer ist als dieser;
wobei der erste Druck kleiner ist als der zweite Druck, so dass das Betätigen des ersten Ventilelements (70) durch das ausgeatmete Gas einem Betreuer einen Hinweis auf relativ schwache Ausatmungen der Person (12) gibt und das Betätigen des zweiten Ventilelements (72) durch das ausgeatmete Gas in Kombination mit dem Betätigen des ersten Ventilelements (70) während stärkerer Ausatmungen der Person (12) auftritt, um den Widerstand gegen die Gasströmung durch die eine oder mehrere Abluftöffnungen (66, 68) während relativ starker Ausatmungen zu reduzieren.

2. Maske (10) nach Anspruch 1, wobei der Widerstand der Maske (10) gegen die Strömung des ausgeatmeten Gases in die Atmosphäre bei dem zweiten Druck durch Betätigen des zweiten Ventilelements (72) erheblich reduziert wird.

3. Maske (10) nach Anspruch 1, wobei das erste Ventilelement (70) und das zweite Ventilelement (72) Teile der gleichen Abluftöffnung (50) abdecken.

4. Maske (10) nach Anspruch 1, wobei eines oder beide, das erste Ventilelement (70) und/oder das zweite Ventilelement (72), Klappen sind.

5. Maske (10) nach Anspruch 1, wobei eines oder beide, das erste Ventilelement und/oder das zweite Ventilelement (70, 72), betätigt werden, um Teile der einen oder mehreren Abluftöffnungen (66, 68) durch Schwenken freizulegen.

6. Maske (10) nach Anspruch1, wobei die eine oder mehrere Abluftöffnungen an dem Quellenkonnektor (18) des Maskenkörpers (14) ausgebildet sind.

## Revendications

1. Masque (10) permettant en grande partie de sceller les voies respiratoires d'un sujet (12) pour lui délivrer une substance respirable, le masque (10) comprenant :
un corps de masque (14) configuré pour envelopper un ou plusieurs orifices externes des voies respiratoires du sujet (12), le corps du masque (14) et le visage (22) du sujet (12) formant une chambre (24) autour du ou des orifices externes des voies respiratoires du sujet (12) qui sont enveloppés par le corps du masque (14), le corps du masque (14) comprenant en outre un connecteur de source (18) configuré pour connecter une source de substance respirable à la chambre (24) de telle sorte que la substance respirable puisse être introduite dans la chambre (24) à partir d'une source de la substance respirable à travers le connecteur de source (18) ;
une ou des soupapes d'échappement (66, 68) formées dans le corps de masque (14) qui laissent le gaz de la chambre (24) s'échapper dans l'atmosphère ambiante ;
un premier élément de valve (70) qui couvre une portion de la ou des soupapes d'échappement (66, 68) pour empêcher le gaz présent dans l'atmosphère ambiante de pénétrer dans la chambre (24) via la ou les soupapes d'échappement (66, 68), le premier élément de valve (70) étant configuré de telle sorte que l'expiration de gaz par le ou les orifices externes des voies respiratoires du sujet (12) engendre une force qui actionne le premier élément de valve (70) pour découvrir la portion de la ou des soupapes d'échappement (66, 68) couvertes par le premier élément de valve (70) si la pression du gaz expiré dans la chambre (24) est égale ou supérieure à une première pression ; et
un deuxième élément de valve (72) qui couvre une portion de la ou des soupapes d'échappement (66, 68) pour empêcher le gaz présent dans l'atmosphère ambiante de pénétrer dans la chambre (24) via la ou les soupapes d'échappement (66, 68), le deuxième élément de valve (72) étant configuré de telle sorte que l'expiration de gaz par le ou les orifices externes des voies respiratoires du sujet (12) engendre une force qui actionne le deuxième élément de valve (72) pour découvrir la portion de la ou des soupapes d'échappement (66, 68) couvertes par le deuxième élément de valve (72), le deuxième élément de valve (72) étant actionné pour découvrir la portion de la ou des soupapes d'échappement (66, 68) couvertes par le deuxième élément de valve (72) si la pression du gaz expiré dans la chambre (24) est égale ou supérieure à une deuxième pression ;
dans lequel la première pression est inférieure à la deuxième pression, de telle sorte que l'actionnement du premier élément de valve (20) par le gaz expiré indique au personnel soignant que les expirations du sujet (12) sont relativement faibles et l'actionnement du deuxième élément de valve (72) par le gaz expiré est combiné à l'actionnement du premier élément de valve (70) lors d'expirations plus fortes de la part du sujet (12) pour réduire la résistance aux arrivées de gaz par la ou les soupapes d'échappement (66, 68) lors d'expirations relativement fortes.

2. Masque (10) selon la revendication 1, dans lequel la résistance du masque (10) à la fuite du gaz expiré dans l'atmosphère est réduite significativement à la seconde pression par l'actionnement du deuxième élément de valve (72).

3. Masque (10) selon la revendication 1, dans lequel le premier élément de valve (70) et le deuxième élément de valve (72) couvrent des portions de la même soupape d'échappement (50).

4. Masque (10) selon la revendication 1, dans lequel le premier élément de valve (70) et/ou le deuxième élément de valve (72) sont rabattables.

5. Masque (10) selon la revendication 1, dans lequel le premier élément de valve et/ou le deuxième élément de valve (70,72) sont actionnés pour couvrir des portions de la ou des soupapes d'échappement (66, 68) en pivotant.

6. Masque (10) selon la revendication 1, dans lequel la ou les soupapes d'échappement sont formées sur le connecteur de source (18) du corps de masque (14).
